# EUROPEAN PATENT APPLICATION

(11) **EP 2 896 349 A1**
(43) Date of publication of application: **22.07.2015**
(21) Application number: 13837177.8
(22) Date of filing: 06.09.2013
(51) Int. Cl.: A61B 1/00, A61B 1/04

(54) **MEDICAL SYSTEM**

(30) Priority: 11.09.2012 JP 2012199545
(71) Applicant: Olympus Corporation, Shibuya-ku Tokyo 151-0072 (JP)
(72) Inventor: IWAISAKO, Hiroshi, Tokyo 151-0072 (JP)
(74) Representative: Winter, Brandl, Fürniss, Hübner, Röss, Kaiser, Polte - Partnerschaft mbB
(86) International application number: PCT/JP2013/074101
(87) International publication number: WO 2014/042096

(57) **Abstract**

By providing an in-vitro apparatus 30 arranged outside a body of an examinee 100 with a judgment section 35 which judges whether a capsule endoscope 1 is in a predetermined digestive tract or not by analysis of an image transmitted from the capsule endoscope 1 and a signal control section 36 which performs output control of a control signal for causing an image acquisition section (an illumination section 2 and an image pickup section 3) and a wireless transmission section 4 of the capsule endoscope 1 to continuously operate or intermittently operate, according to a judgment result of the judgment section 35, exhaustion of the power source section 8 of the capsule endoscope 1 is suppressed and a desired image is obtained, without imposing a burden on an observer.

## Description

### Technical Field

The present invention relates to a medical system capable of acquiring in-vivo image information using a capsule endoscope.

### Background Art

Endoscopes in a medical field have been conventionally used for a purpose of in-vivo observation and the like. As one of the endoscopes, a capsule endoscope has been proposed recently which is capable of being arranged in a body cavity by being swallowed by an examinee, picking up an image of an object while moving in the body cavity accompanying peristaltic movement, and wirelessly transmitting the picked-up image of the object to an outside as an image pickup signal.

As a capsule endoscope of this type, for example, Japanese Patent Application Laid-Open Publication No. 2009-89907 discloses a configuration capable of, by radiating an alternating magnetic field from an outside of the capsule endoscope, switching start and stop of the capsule endoscope. That is, a living body observation system disclosed in Japanese Patent Application Laid-Open Publication No. 2009-89907 is provided with a capsule endoscope configured having such dimensions, a form and the like that enable arrangement in a living body, and a magnetic field generating section causing an alternating magnetic field to occur outside the capsule endoscope. The magnetic field generating section has a configuration capable of switching a magnetic field generation state to either on or off, for example, according to a switch operation or the like by a user. On the other hand, the capsule endoscope internally has an illumination section which emits an illuminating light for illuminating an object in a living body; an image pickup section which picks up an image of the object illuminated by the illumination section and outputs the image as an image pickup signal; a wireless transmission section which wirelessly transmits the image pickup signal outputted from the image pickup section outside the living body; a power supply section which supplies driving power required to drive each of the illumination section, the image pickup section and the wireless transmission section; and a magnetic field detecting section capable of detecting an alternating magnetic field emitted by the magnetic field generating section. By the above configuration, it is possible to switch between on and off of power each time the alternating magnetic field generated by the magnetic field generating section is detected.

Furthermore, Japanese Patent Application Laid-Open Publication No. 2009-89907 discloses a technique for, after arranging a capsule endoscope in a body of an examinee, switching between on and off of a power source by an alternating magnetic field generated from the magnetic field generation section, aiming at suppressing exhaustion of a built-in battery. More specifically, for example, such control is possible that the power source is off while the capsule endoscope is passing through a site for which observation and the like are not necessary, and when the capsule endoscope reaches a desired observation site, the power source of the capsule endoscope is on by causing an alternating magnetic field to be generated from the magnetic field generating section. Furthermore, when the capsule endoscope reaches a site for which observation and the like are not necessary, the power source of the capsule endoscope can be turned off by generating an alternating magnetic field from the magnetic field generating section again.

However, as disclosed in Japanese Patent Application Laid-Open Publication No. 2009-89907 described above, it is necessary for a doctor or a technician, who is an observer, to directly observe a monitor while the capsule endoscope is moving in a body in order to turn on the power source when the capsule endoscope in the body reaches a desired site or turn off the power source when the capsule endoscope has passed through a desired site. This is a large burden on the observer.

The present invention has been made in view of the above situation, and an object of the invention is to provide a medical system capable of suppressing exhaustion of a power supply and obtaining a desired image without imposing a burden on an observer.

### Disclosure of Invention

### Means for Solving the Problem

A medical system according to an aspect of the present invention includes: a capsule endoscope including an image acquisition section acquiring an image, an image signal transmitting section wirelessly transmitting the image, a power source section capable of supplying power to the image acquisition section and the image signal transmitting section, a control signal receiving section receiving a control signal transmitted from an outside, and a switch section switching between on and off states of power supply from the power source section to the image acquisition section and the image signal transmitting section according to the control signal, the capsule endoscope being to be swallowed by an examinee; and an in-vitro apparatus including an image signal receiving section receiving the image signal, a judgment section judging whether the capsule endoscope is in a predetermined digestive tract or not by analysis of the image, and a signal control section performing output control of the control signal for causing the image acquisition section and the image signal transmitting section to continuously operate or intermittently operate according to a judgment result of the judgment section, the in-vitro apparatus being arranged outside the examinee.

### Brief Description of the Drawings

Fig. 1 relates to a first embodiment of the present invention and is a schematic configuration diagram of a medical system used for an examinee;
Fig. 2 relates to the first embodiment of the present invention and is a functional block diagram showing main sections of a capsule endoscope;
Fig. 3 relates to the first embodiment of the present invention and is a functional block diagram showing main sections of an in-vitro apparatus;
Fig. 4 relates to the first embodiment of the present invention and is a flowchart showing an operation mode control routine;
Fig. 5 relates to the first embodiment of the present invention and is a diagram schematically showing an example of an image picked up in a stomach;
Fig. 6 relates to the first embodiment of the present invention and is a diagram schematically showing an example of an image picked up in a duodenal bulb;
Fig. 7 relates to the first embodiment of the present invention and is a diagram schematically showing an example of an image picked up in a small intestine;
Fig. 8 relates to the first embodiment of the present invention and is a flowchart showing a first modification of the operation mode control routine;
Fig. 9 relates to the first embodiment of the present invention and is a flowchart showing a second modification of the operation mode control routine; and
Fig. 10 relates to a second embodiment of the present invention and is a functional block diagram showing main sections of a capsule endoscope.

### Best Mode for Carrying Out the Invention

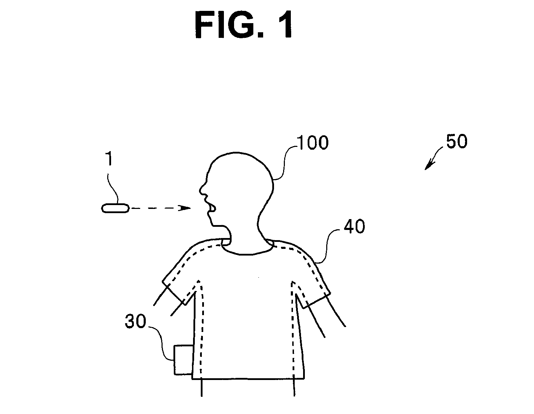

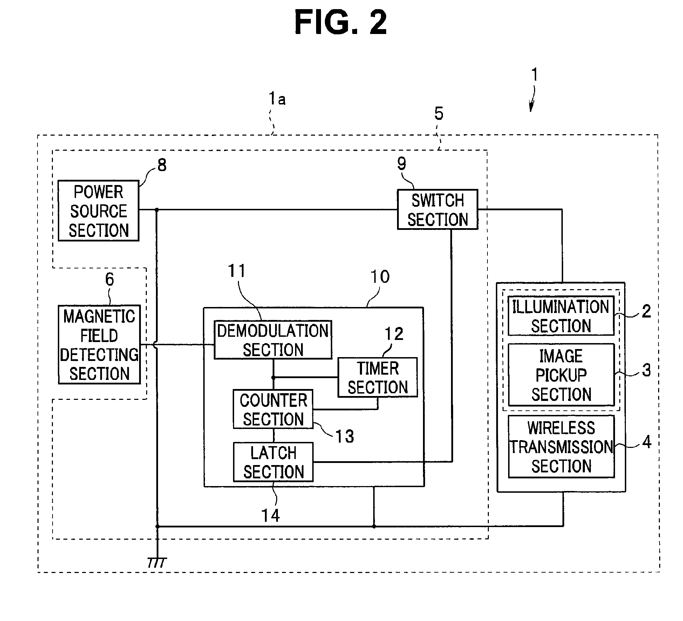

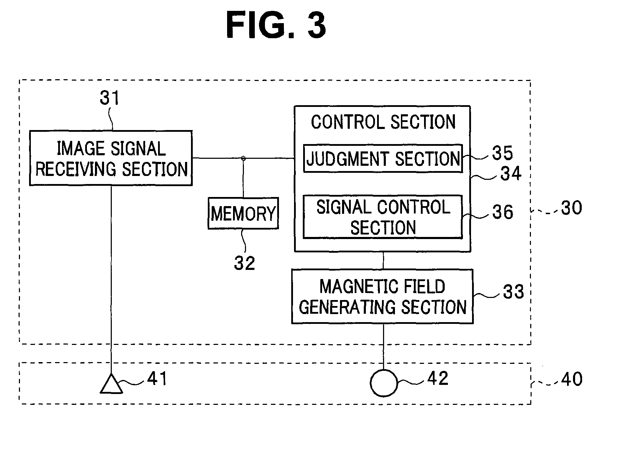

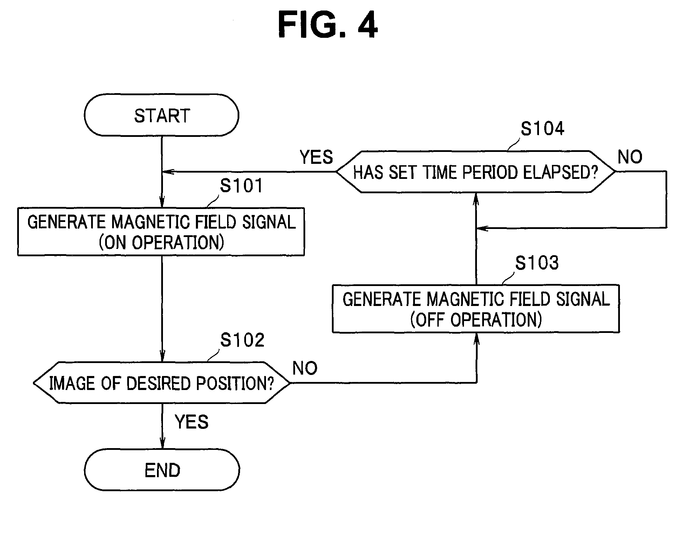

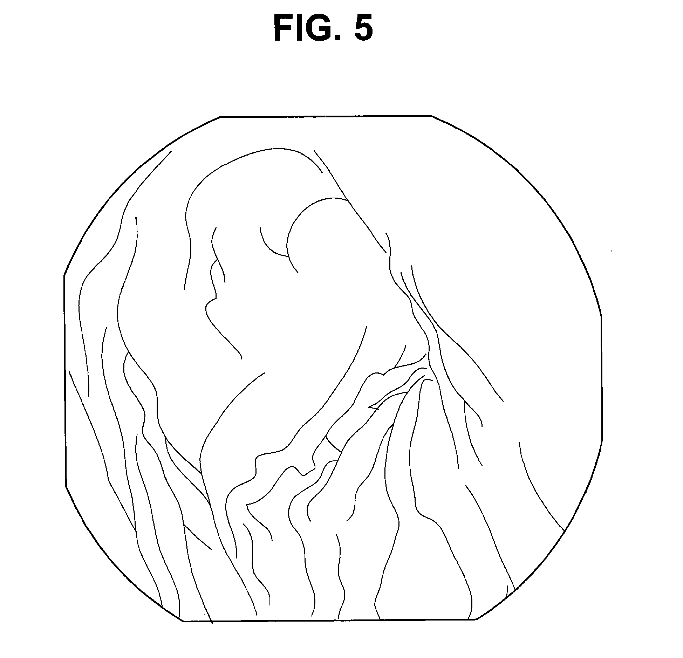

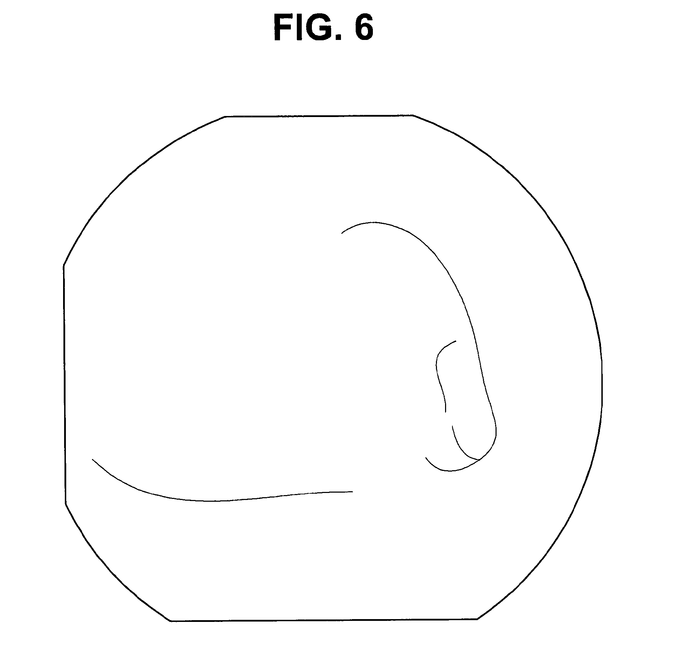

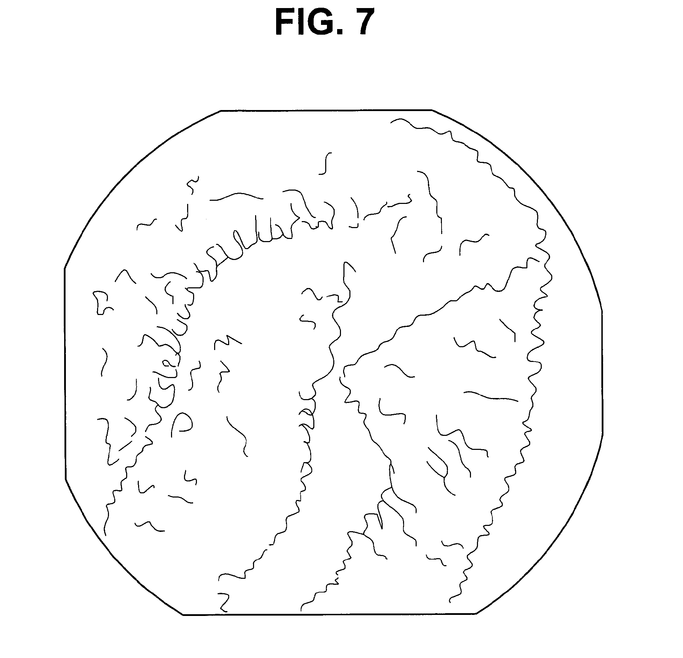

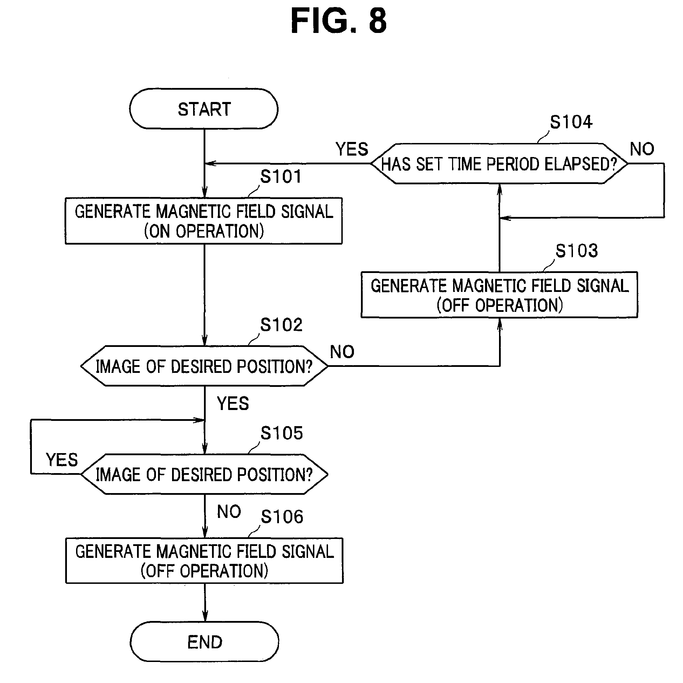

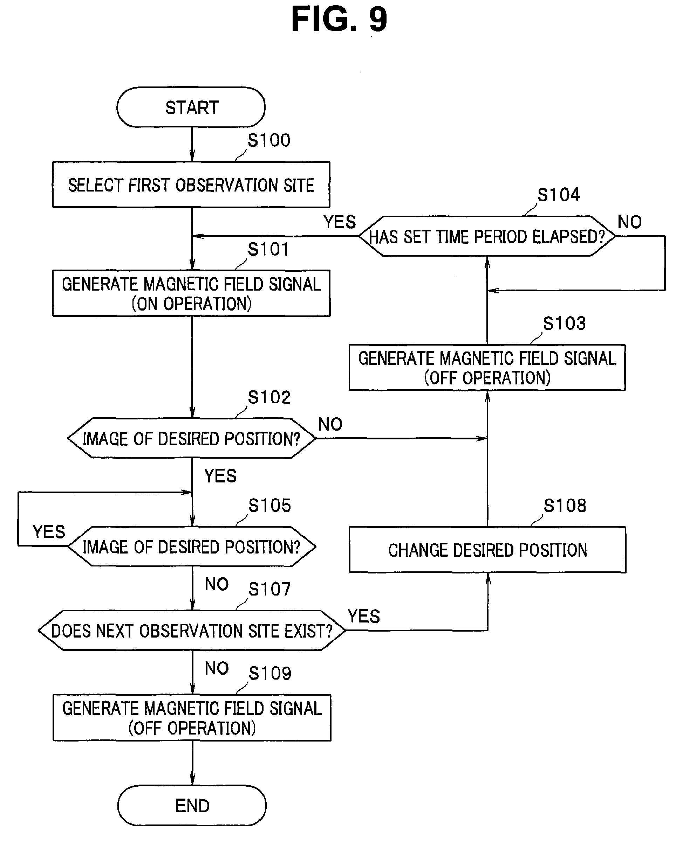

Embodiments of the present invention will be described below with reference to drawings. Figs. 1 to 9 relate to a first embodiment of the present invention. Fig. 1 is a schematic configuration diagram of a medical system used for an examinee; Fig. 2 is a functional block diagram showing main sections of a capsule endoscope; Fig. 3 is a functional block diagram showing main sections of an in-vitro apparatus; Fig. 4 is a flowchart showing an operation mode control routine; Fig. 5 is a diagram schematically showing an example of an image picked up in a stomach; Fig. 6 is a diagram schematically showing an example of an image picked up in a duodenal bulb; Fig. 7 is a diagram schematically showing an example of an image picked up in a small intestine; Fig. 8 is a flowchart showing a first modification of the operation mode control routine; and Fig. 9 is a flowchart showing a second modification of the operation mode control routine.

A medical system 50 shown in Fig. 1 is provided with a swallow capsule endoscope 1 introduced into a body cavity of an examinee 100, and an in-vitro apparatus 30 arranged outside the examinee 100 to wirelessly communicate various information with the capsule endoscope 1.

As shown in Fig. 2, the capsule endoscope 1 internally has an illumination section 2 which emits an illuminating light for illuminating an object in the body cavity of the examinee 100; an image pickup section 3 which picks up an image of the object illuminated by the illumination section to acquire image data; a wireless transmission section 4 as an image signal transmitting section which modulates the image data acquired by the image pickup section 3 into a wireless signal and transmits the wireless signal to an outside; a power supply section 5 capable of supplying driving power for driving each of the illumination section 2, the image pickup section 3 and the wireless transmission section 4; and a magnetic field detecting section 6 capable of detecting a magnetic field (for example, a burst alternating magnetic field generated multiple times) generated by the in-vitro apparatus 30 inside a case 1a. That is, an image acquisition section in the present embodiment is configured, for example, being provided with the illumination section 2 and the image pickup section 3.

The power supply section 5 is configured having a power source section 8 constituted by a built-in battery or the like capable of supplying power for driving each section of the capsule endoscope 1, a switch section 9 and a signal receiving section 10.

The magnetic field detecting section 6 is configured, for example, with a resonance circuit constituted by a coil and a capacitor. The magnetic field detecting section 6 generates a magnetic field detection signal, which is an electrical signal corresponding to a detection result of an alternating magnetic field emitted from the in-vitro apparatus 30 (for example, a waveform and the like at a time of detection) and outputs the magnetic field detection signal to the signal receiving section 10.

The power source section 8 is connected such that it continuously supplies power to the signal receiving section 10 and supplies power to each of the illumination section 2, the image pickup section 3 and the wireless transmission section 4 via the switch section 9.

The switch section 9 is connected between each of the illumination section 2, the image pickup section 3 and the wireless transmission section 4, and the power source section 8. The switch section 9 is configured such that the switch section 9 can switch a state of power supply from the power source section 8 to each of the illumination section 2, the image pickup section 3 and the wireless transmission section 4 to a continuity state or an open state by being turned on or off on a basis of an output state of a switching signal from the signal receiving section 10.

The signal receiving section 10 is configured having a demodulation section 11 which demodulates a magnetic field detection signal having a signal level equal to or higher than a threshold set in advance and outputs a pulse signal; a timer section 12 which measures a time period until a predetermined time period elapses after the demodulation section 11 starts to output the pulse signal; a counter section 13 which obtains a count value by counting the number of inputs of the pulse signal from the demodulation section 11 one by one; and a latch section 14 which reverses output of a switching signal for switching between on and off states of the switch section 9 each time an output signal from the counter section 13 is inputted.

The demodulation section 11 is configured, for example, with a peak hold circuit constituted by a diode, a capacitor and a resistance. That is, the demodulation section 11 is configured such that the demodulation section 11 can appropriately demodulate a magnetic field detection signal outputted from the magnetic field detecting section 6 in a case where an output interval between magnetic field detection signals outputted from the magnetic field detecting section 6 is equal to or more than a time constant τ which is determined by a product of a capacity of the capacitor and a value of the resistance. The demodulation section 11 is configured such that the demodulation section 11 can appropriately demodulate a magnetic field detection signals outputted from the magnetic field detecting section 6 by demodulating a magnetic field detection signal outputted from the in-vitro apparatus 30 at each output interval equal to or more than the time constant τ. The demodulation section 11 is configured such that the demodulation section 11 can continuously output the number of pulse signals corresponding to the number of outputs of an alternating magnetic field which is outputted from the in-vitro apparatus 30 in a burst state, to the counter section 13 by demodulating a magnetic field detection signal outputted from the magnetic field detecting section 6 at each output interval equal to or more than the time constant τ.

The timer section 12 operates so that the count value of the counter section 13 is reset to 0 when the predetermined time period has elapsed.

The counter section 13 is configured so as to, when detecting that the count value obtained by counting the number of inputs of the pulse signal from the demodulation section 11 one by one has reached a signal output count value, output an output signal to the latch section 14.

As shown in Fig. 3, the in-vitro apparatus 30 is configured having an image signal receiving section 31, a memory 32, a magnetic field generating section 33 and a control section 34.

The image signal receiving section 31 receives a wireless signal transmitted from the wireless transmission section 4 of the capsule endoscope 1 via a receiving antenna 41 and extracts image data (endoscopic image data) from the received wireless signal.

The memory 32 is configured, for example, with an external memory or the like, and the memory 32 stores image data extracted by the image signal receiving section 31.

The magnetic field generating section 33 has a configuration capable of generating a burst alternating electric field for switching a power source state in the capsule endoscope 1. More specifically, the magnetic field generating section 33 causes a burst alternating magnetic field to be generated multiple times in response to an output instruction from the control section 34 and transmits the burst alternating magnetic field to the capsule endoscope 1 via a transmitting antenna 42.

The control section 34 is configured having a judgment section 35 and a signal control section 36.

The judgment section 35 judges whether the capsule endoscope 1 is in a predetermined digestive tract or not by image analysis. Here, for example, in a case of detecting a pixel having a predetermined or larger luminance difference from an adjoining pixel as an edge, in comparison among respective images of a stomach, a duodenal bulb and a small intestine, as shown in Figs. 5 to 7, the greatest number of edges can be detected from the image of the small intestine where fine villi exist. In comparison, edges cannot be detected from the image of the substantially flat duodenal bulb almost at all. From the image of the stomach where folds with a certain length extend, continuous edges with the certain length can be detected. For example, when dispersion of luminance in a predetermined area set on the images is analyzed, the luminance dispersion is largest in the small intestine where fine villi exist, second largest in the stomach, and smallest in the duodenal bulb. For example, by performing image analysis on a basis of information obtained by compiling these as a database in advance, it becomes possible to judge movement from the stomach to the duodenal bulb, and movement from the duodenal bulb to the small intestine. As for other digestive tracts, it is possible to discriminate the digestive tracts from various characteristics appearing on an image, though description thereof is omitted.

The signal control section 36 performs output control of a control signal (a burst alternating magnetic field generated multiple times) for causing the image acquisition section (the illumination section 2 and the image pickup section 3) and the wireless transmission section 4 to continuously operate or intermittently operate according to a judgment result of the judgment section 35. For example, when the small intestine is set as an examination target, the signal control section 36 performs output control of a control signal for the capsule endoscope 1 so that the image acquisition section and the wireless transmission section 4 intermittently operate until the judgment section 35 judges that the capsule endoscope 1 has reached the small intestine, and performs output control of the control signal for the capsule endoscope 1 so that the image acquisition section and the wireless transmission section 4 continuously operate after the judgment section 35 judges that the capsule endoscope 1 has reached the small intestine.

Here, for example, as shown in Figs. 1 and 2, the receiving antenna 41 and the transmitting antenna 42 are arranged on pullover clothes (for example, a vest) 40 to be worn on the examinee 100.

Next, description will be made on operation mode control for the capsule endoscope 1 executed by the control section 34 of the in-vitro apparatus 30 in accordance with the flowchart of the operation mode control routine shown in Fig. 4. Note that, for example, control in a case where a small intestine is set as an examination target (a desired position) will be described below.

The routine is executed by the capsule endoscope 1 being swallowed by the examinee 100 and an on operation of a switch of the in-vitro apparatus 30, which is not shown, being performed. When the routine starts, the control section 34 first causes a burst alternating magnetic field to be generated multiple times through the magnetic field generating section 33 so as to cause the switch section 9 of the capsule endoscope 1 to perform an on operation, at step S101.

The alternating magnetic field is transmitted to the capsule endoscope 1 via the transmitting antenna 42 and received at the magnetic field detecting section 6 of the capsule endoscope 1. Then, the alternating magnetic field received at the magnetic field detecting section 6 is converted to a magnetic field detection signal and then outputted to the signal receiving section 10. When the magnetic field detection signal is inputted, the signal receiving section 10 causes the switch section 9 to perform an on operation, and power supply from the power source section 8 to the illumination section 2, the image pickup section 3 and the wireless transmission section 4 is started. Thereby, an object in the body cavity of the examinee 100 is illuminated by the illumination section 2, and an image of the object is picked up by the image pickup section 3. Image data of the object which has been image-picked up is modulated to a wireless signal by the wireless transmission section 4 and transmitted to the in-vitro apparatus 30. The wireless signal from the wireless transmission section 4 is received via the receiving antenna 41. After the received signal is demodulated to the image data at the image signal receiving section 31 of the in-vitro apparatus 30, the image data is stored into the memory 32 and appropriately inputted to the control section 34.

When proceeding from step S101 to step S102, the control section 34 performs analysis processing of newest image data inputted from the image signal receiving section 31 and judges whether the currently acquired image is an image of a desired position in a digestive tract or not (for example, an image of a small intestine or not).

Then, if judging that the currently acquired image is not an image of the desired position as a result of analysis of the image, the control section 34 proceeds to step S103 and causes a burst alternating current signal to be generated multiple times through the magnetic field generating section 33 so as to cause the switch section 9 of the capsule endoscope 1 to perform an off operation.

The alternating magnetic field is transmitted to the capsule endoscope 1 via the transmitting antenna 42 and received by the magnetic field detecting section 6 of the capsule endoscope 1. Then, the alternating magnetic field received at the magnetic field detecting section 6 is converted to a magnetic field detection signal and then outputted to the signal receiving section 10. When the magnetic field detection signal is inputted, the signal receiving section 10 causes the switch section 9 to perform an off operation, and power supply from the power source section 8 to the illumination section 2, the image pickup section 3 and the wireless transmission section 4 is stopped.

When proceeding from step S103 to step S104, the control section 34 checks whether or not a set time period T has elapsed after the off operation of the switch section 9. If judging that the set time period T has not elapsed yet, the control section 34 waits as it is.

On the other hand, if judging that the set time period T has elapsed after the off operation of the switch section 9, at S104, the control section 34 returns to step S101.

Then, by such a series of processes from step S101 to step S104 being performed by the control section 34, the illumination section 2, the image pickup section 3 and the wireless transmission section 4 are caused to intermittently operate for each set time period T in the capsule endoscope 1 (that is, the capsule endoscope 1 is caused to operate in an intermittent operation mode).

If judging that the currently acquired image is an image of the desired position as a result of the image analysis at step S102, the control section 34 exits the routine as it is (that is, in the state that the switch section 9 of the capsule endoscope 1 is caused to be performing the on operation at step S101).

Then, by the operation mode control routine ending while the switch section 9 is caused to be performing the on operation as described above, the illumination section 2, the image pickup section 3 and the wireless transmission section 4 are caused to continuously operate in the capsule endoscope 1 (that is, the capsule endoscope 1 is caused to operate in a continuous operation mode).

According to such an embodiment, by providing the in-vitro apparatus 30 arranged outside the body of the examinee 100 with the judgment section 35 which judges whether the capsule endoscope 1 is in a predetermined digestive tract or not by analysis of an image transmitted from the capsule endoscope 1 and the signal control section 36 which performs output control of a control signal for causing the image acquisition section (the illumination section 2 and the image pickup section 3) and the wireless transmission section 4 of the capsule endoscope 1 to continuously operate or intermittently operate, according to a judgment result of the judgment section 35, it is possible to suppress exhaustion of the power source section 8 of the capsule endoscope 1 and obtain a desired image, without imposing a burden on an observer.

In this case, especially exhaustion of the power source section 8 until a desired site is reached is small, and, therefore, it is possible to appropriately suppress occurrence of a trouble such as photographing being stopped due to occurrence of battery exhaustion during photographing.

Further, since it is not necessary for the observer to check an image for each set time period, a patient (the examinee 100) can freely move.

Next, a first modification of the present embodiment will be described in accordance with the flowchart of the operation mode control routine shown in Fig. 8. Note that, in the present modification, description will be made only on points different from the operation mode control shown in Fig. 4.

If judging that the currently acquired image is an image of the desired position as a result of the image analysis at step S102, the control section 34 proceeds to step S105.

At step S105, the control section 34 performs analysis processing of newest image data inputted from the image signal receiving section 31 by an analysis process similar to step S102 described above, and judges whether the currently acquired image is an image of a desired position in a digestive tract or not (for example, an image of a small intestine or not).

Then, if judging that the currently acquired image is an image of the desired position as a result of the image analysis at step S105, the control section 34 waits as it is, and repeatedly executes the analysis process each time newest image data is inputted.

On the other hand, if judging that the currently acquired image is not an image of the desired position any more at step S105, the control section 34 proceeds to step S106 and causes a burst alternating current signal to be generated multiple times through the magnetic field generating section 33 so as to cause the switch section 9 of the capsule endoscope 1 to perform an off operation and, after that, exits the routine.

According to such a modification, an effect is obtained that it is possible to, by picking up an image only of a desired position, perform image checking work after acquisition without waste, in addition to the effect described above.

Next, a second modification of the present embodiment will be described in accordance with the flowchart of the operation mode control routine shown in Fig. 9. The present modification is for acquiring images for multiple desired sites. Description will be made, for example, on a case of acquiring images of a stomach and a small intestine as the desired sites as an example. Note that, in the present modification, description will be made only on points different from the operation mode control shown in Fig. 8.

When the routine starts, the control section 34 selects a first observation site as a desired position prior to the process of step 8101. That is, for example, in the present modification in which images for the stomach and the small intestine are to be acquired, the stomach is selected as a desired position.

If judging that the currently acquired image is not an image of the desired position (in the present modification, an image of the currently selected stomach or small intestine) any more at step S105 of the present routine, the control section 34 proceeds to step S107 and checks whether or not a next site exists as a desired observation site. That is, for example, in the present modification in which images for the stomach and the small intestine are to be acquired, it is checked whether or not the small intestine has not been selected yet.

Then, when it is judged that the next observation site exists at step S107, the control section 34 proceeds to step S108 and changes the desired position to the next observation site (for example, changes the desired position from the stomach to the small intestine). After that, the control section 34 returns to step S103.

On the other hand, if it is judged that the next observation site does not exist at step S107, the control section 34 proceeds to step S109 and causes a burst alternating current signal to be generated multiple times through the magnetic field generating section 33 so as to cause the switch section 9 of the capsule endoscope 1 to perform an off operation and, after that, exits the routine.

According to such a modification, an effect is obtained that it is possible to appropriately acquire an image of each observation site even in a case where multiple desired observation sites exist, in addition to each effect described above.

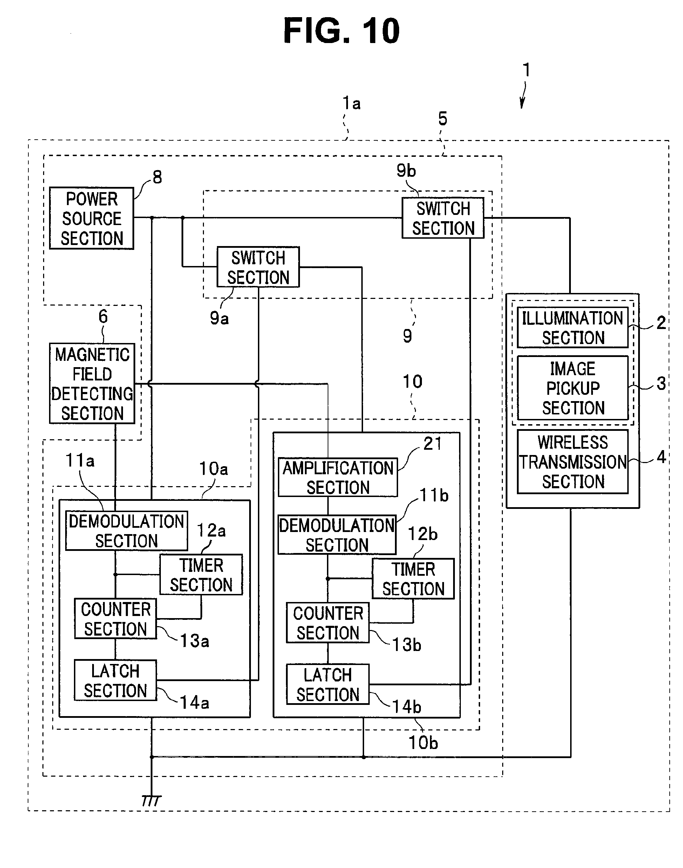

Next, Fig. 10 relates to a second embodiment of the present invention, and Fig. 10 is a functional block diagram showing main sections of a capsule endoscope. Note that the present embodiment is mainly different from the first embodiment described above in that, for example, the switch section 9 is configured with two switch sections 9a and 9b, and the signal receiving section 10 is configured with two signal receiving sections 10a and 10b. Additionally, as for components and the like similar to those in the first embodiment described above, same reference numerals are given, and description thereof is omitted.

As shown in Fig. 10, the switch section 9a is connected to the power source section 8 and is configured such that the switch section 9a can switch the state of power supply from the power source section 8 to the signal receiving section 10b to the continuity state or the open state by being turned on or off on a basis of an output state of a switching signal from the signal receiving section 10a provided with a function as a first switching control section.

The switch section 9b is connected between each of the illumination section 2, the image pickup section 3 and the wireless transmission section 4, and the power source section 8. The switch section 9b is configured such that the switch section 9b can switch the state of power supply from the power source section 8 to each of the illumination section 2, the image pickup section 3 and the wireless transmission section 4 to the continuity state or the open state by being turned on or off on a basis of an output state of a switching signal from the signal receiving section 10b provided with a function as a second switching control section.

The signal receiving section 10a is configured having a demodulation section 11 a which demodulates a magnetic field detection signal having a signal level equal to or higher than a threshold TH1 and outputs apulse signal; a timer section 12a which measures a time period until a predetermined time period TA1 elapses after the demodulation section 11a starts to output the pulse signal; a counter section 13a which obtains a count value by counting the number of inputs of the pulse signal from the demodulation section 11a one by one; and a latch section 14a which reverses output of a switching signal for switching between on and off states of the switch section 9a each time an output signal from the counter section 13a is inputted.

The demodulation section 11 a is configured, for example, with a peak hold circuit constituted by a diode, a capacitor and a resistance. That is, the demodulation section 11 a is configured such that the demodulation section 11 a can appropriately demodulate a magnetic field detection signal outputted from the magnetic field detecting section 6 in a case where an output interval between magnetic field detection signals outputted from the magnetic field detecting section 6 is equal to or more than a time constant τ1 which is determined by a product of a capacity of the capacitor and a resistance value of the resistance. The demodulation section 11 a is configured such that the demodulation section 11a can continuously output the number of pulse signals corresponding to the number of outputs of an alternating magnetic field which is outputted from the magnetic field generating section 33 (the number of outputs of the magnetic field detection signal outputted from the magnetic field detecting section 6) in a burst state, to the counter section 13a by demodulating a magnetic field detection signal outputted from the magnetic field detecting section 6 at each output interval equal to or more than the time constant τ1.

The timer section 12a operates so that the count value of the counter section 13a is reset to 0 when the predetermined time period TA1 has elapsed.

Note that the timer section 12a of the present embodiment is not limited to one that measures a time period until the predetermined time period TA1 elapses after the demodulation section 11a starts to output a pulse signal. For example, one that measures a time period corresponding to an output interval between pulse signals outputted from the demodulation section 11a is also possible, or one that performs the two time period measurements at a same time is also possible. Further, in a case where the timer section 12a is configured so as to measure a time period corresponding to the output interval between pulse signals outputted from the demodulation section 11a, the time constant τ1 described before may substantially correspond to the measurement time period of the timer section 12a.

The counter section 13a is configured so as to, when detecting that the count value obtained by counting the number of inputs of the pulse signal from the demodulation section 11a one by one has reached a signal output count value PA, output an output signal to the latch section 14a.

On the other hand, the signal receiving section 10b is configured having an amplification section 21 which amplifies a signal level of a magnetic field detection signal outputted from the magnetic field detecting section 6 to a signal level equal to or higher than a threshold TH2, a demodulation section 11b which demodulates a magnetic field detection signal having a signal level equal to or higher than the threshold TH2 and outputs a pulse signal; a timer section 12b which measures a time period until a predetermined time period TA2 elapses after the demodulation section 11b starts to output the pulse signal; a counter section 13b which obtains a count value by counting the number of inputs of the pulse signal from the demodulation section 11b one by one; and a latch section 14b which reverses output of a switching signal for switching between on and off states of the switch section 9b each time an output signal from the counter section 13b is inputted.

Here, the signal receiving section 10b of the present embodiment is set so that a detecting sensitivity of an alternating magnetic field (a control signal) generated by the magnetic field generating section 33 is relatively higher than that of the signal receiving section 10a, by being provided with the amplification section 21.

The demodulation section 11 b is configured, for example, with a peak hold circuit constituted by a diode, a capacitor and a resistance. That is, the demodulation section 11b is configured such that the demodulation section 11b can appropriately demodulate a magnetic field detection signal outputted from the magnetic field detecting section 6 in a case where an output interval between magnetic field detection signals outputted from the magnetic field detecting section 6 is equal to or more than a time constant τ2 which is determined by a product of a capacity of the capacitor and a resistance value of the resistance. The demodulation section 11a is configured such that the demodulation section 11a can continuously output the number of pulse signals corresponding to the number of outputs of an alternating magnetic field which is outputted from the magnetic field generating section 33 (the number of outputs of the magnetic field detection signal outputted from the magnetic field detecting section 6) in a burst state, to the counter section 13b by demodulating a magnetic field detection signal outputted from the magnetic field detecting section 6 at each output interval equal to or more than the time constant τ2.

The timer section 12b operates so that the count value of the counter section 13b is reset to 0 when the predetermined time period TA2 has elapsed.

Note that the timer section 12b of the present embodiment is not limited to one that measures a time period until the predetermined time period TA2 elapses after the demodulation section 11b starts to output a pulse signal. For example, one that measures a time period corresponding to an output interval between pulse signals outputted from the demodulation section 11b is also possible, or one that performs the two time period measurements at a same time is also possible. Further, in a case where the timer section 12b is configured so as to measure a time period corresponding to the output interval between pulse signals outputted from the demodulation section 11b, the time constant τ2 described before may correspond to the measurement time period of the timer section 12b.

According to the present embodiment, the predetermined time periods TA1 and TA2 described before may be set to a same time period or may be set so that TA1>TA2 is satisfied.

The counter section 13b is configured so as to, when detecting that the count value obtained by counting the number of inputs of the pulse signal from the demodulation section 11b one by one has reached a signal output count value PB, output an output signal to the latch section 14b.

Note that each of the signal output count values PA and PB described before may be set to an arbitrary value as far as a magnitude relationship of PA>PB is satisfied.

In the present embodiment, it is assumed that the capacity of each capacitor and the resistance value of each resistance are set so that the time constant τ1 of the demodulation section 11a and the time constant τ2 of the demodulation section 11b are same values.

In such an embodiment, it is possible to perform control similar to each of the operation mode controls shown in Figs. 4, 8 and 9 in the first embodiment described above. In the present embodiment, however, an on operation of the switch section 9a is performed before the capsule endoscope 1 is swallowed into the examinee 100, and the on operation or the off operation by generation of a magnetic field signal at steps S101, S103, S106 and S109 described above are performed with the switch section 9b as a target.

Note that, a magnetic field for causing the switch section 9a to perform an on/off operation can be generated by the magnetic field generating section 33 of the in-vitro apparatus 30. Alternatively, the magnetic field for causing the switch section 9a to perform an on/off operation may be generated by a magnetic field generating section provided separately from the magnetic field generating section 33.

According to such an embodiment, operations and effects similar to those of the first embodiment described above can be obtained. In addition, in the present embodiment, even in a case where only a weak magnetic field can be detected by the magnetic field detecting section 6 in the examinee 100, it is possible to appropriately switch the state of power supply from the power source section 8 to the illumination section 2, the image pickup section 3 and the wireless transmission section 4 to the continuity state or the open state without increasing the size of the magnetic field detecting section 6, due to the operation of the amplification section 21. In this case, since power is not supplied from the power source section 8 to the amplification section 21 while the switch section 9a is off, it is possible to appropriately suppress unnecessary power consumption.

Note that the present invention is not limited to each embodiment described above. Various modifications and changes are possible, and the modifications and changes are also within a technical range of the present invention.

This application is filed claiming priority based on Japanese Patent Application No. 2012-199545 filed on September 11, 2012, the contents of which is incorporated in the specification, claims and drawings of the present application by reference thereto.

## Claims

1. A medical system comprising:
a capsule endoscope including an image acquisition section acquiring an image, an image signal transmitting section wirelessly transmitting the image, a power source section capable of supplying power to the image acquisition section and the image signal transmitting section, a control signal receiving section receiving a control signal transmitted from an outside, and a switch section switching between on and off states of power supply from the power source section to the image acquisition section and the image signal transmitting section according to the control signal, the capsule endoscope being to be swallowed by an examinee; and
an in-vitro apparatus including an image signal receiving section receiving the image signal, a judgment section judging whether the capsule endoscope is in a predetermined digestive tract or not by analysis of the image, and a signal control section performing output control of the control signal for causing the image acquisition section and the image signal transmitting section to continuously operate or intermittently operate according to a judgment result of the judgment section, the in-vitro apparatus being arranged outside the examinee.

2. The medical system according to claim 1, wherein the signal control section causes the image acquisition section and the image signal transmitting section to intermittently operate when it is judged that the capsule endoscope is not in the predetermined digestive tract, and causes the image acquisition section and the image signal transmitting section to continuously operate when it is judged that the capsule endoscope is in the predetermined digestive tract.
